(19) Europäisches Patentamt
European Patent Office
Office européen des brevets

(11) Veröffentlichungsnummer: **0 005 805**
A1

(12) **EUROPÄISCHE PATENTANMELDUNG**

(21) Anmeldenummer: 79101606.6

(22) Anmeldetag: 25.05.79

(51) Int. Cl.²: **A 61 K 7/06**
**A 61 K 7/08, A 61 K 7/11**

(30) Priorität: 01.06.78 DE 2823968

(43) Veröffentlichungstag der Anmeldung:
12.12.79 Patentblatt 79/25

(84) Benannte Vertragsstaaten:
BE CH FR GB IT NL SE

(71) Anmelder: Henkel Kommanditgesellschaft auf Aktien
Postfach 1100
D-4000 Düsseldorf 1(DE)

(72) Erfinder: Cortekar, Hans-Wolfgang
Elisabethstrasse 38
D-4018 Langenfeld-Richrath(DE)

(54) Verwendung von Borsäure zur Verbesserung der Struktur und der Nasskämmbarkeit von Haaren.

(57) Gegenstand der Erfindung ist die Verwendung von Borsäure in Haarkonditionierungsmitteln zur gleichzeitigen Verbesserung der Struktur und der Naßkämmbarkeit der Haare.

EP 0 005 805 A1

0005805

HENKEL KGaA
ZR-FE/Patente

z-sü

4000 Düsseldorf, den
Henkelstraße 67

Patentanmeldung

D 5799

"Verwendung von Borsäure zur Verbesserung der Struktur
und der Naßkämmbarkeit von Haaren"

Gegenstand der Erfindung ist die Verbesserung der Struktur und der Naßkämmbarkeit von Haaren, insbesondere von Haaren, die durch haarkosmetische Behandlungen wie Färbungen, Entfärbungen, Dauerwellen nachteilig beeinflußt wurden, durch die Behandlung mit haarkosmetischen Mitteln die Borsäure enthalten.

Als Folge der häufigen Behandlung des Haares mit mehr oder weniger stark schädigenden Mitteln beim Bleichen, Dauerwellen und gegebenenfalls auch Färben tritt neben einer Strukturschädigung auch eine zunehmende Verwirrung der Haare ein, die vor allem bei vollem Haar zu einer wesentlichen Verschlechterung der Naßkämmbarkeit führt. Darüber hinaus bekommt das Haar vielfach ein stumpfes, glanzloses Aussehen und einen verschlechterten Griff.

/2

0005805
HENKEL KGaA
ZR-FE/Patente

Um diesem Übelstand zu begegnen, hat man den haarkosmetischen Präparaten bereits strukturverbessernde
Mittel und Produkte zur Beeinflussung der Naßkämmbarkeit zugesetzt. Als Mittel zur Verbesserung der
Haarstruktur wurden in erster Linie Formaldehyd sowie
formaldehydabspaltende Verbindungen, ferner S-Acetylsuccinanhydrid, Ammoniumvinylphosphonat und andere
eingesetzt. Der Verbesserung der Kämmbarkeit diente in
erster Linie der Zusatz von Fetten, höheren Kohlenwasserstoffen in Frisiercremes und Brillantinen und
von quartären Ammoniumverbindungen. Um sowohl eine
strukturverbessernde Wirkung als auch eine verbesserte
Naßkämmbarkeit zu erreichen, bedurfte es des Zusatzes
mehrerer Mittel, da mit einem Produkt eine Beeinflussung
in beiden Richtungen nicht zu erreichen war.

Es wurde nun gefunden, daß sowohl eine strukturverbessernde Wirkung als auch eine verbesserte Naßkämmbarkeit zu erzielen ist, wenn man in den haarkosmetischen
Mitteln Borsäure einsetzt.

Der Einsatz der Borsäure kann in Frisierlotionen, Frisiercremes, Frisiergelen, Antischuppenlotionen,
Wasserwellmitteln, Haarspülungen, Tönungswäschen,
Fönwellen und ähnlichen Mitteln erfolgen.

Durch die Behandlung des Haares mit den erfindungsgemäßen, Borsäure enthaltenden Mitteln wird erreicht,
daß sich das feuchte Haar wesentlich leichter kämmen
läßt. Darüber hinaus zeichnet sich das trockene Haar
durch einen besseren Glanz, Fülle, angenehmen Griff
aus und zeigt keine Neigung zur statischen Aufladung
beim Bürsten oder Kämmen. Dies gilt sowohl für normales
Haar als auch insbesondere für Haar, das einer Behandlung
mit schädigenden Mitteln beim Bleichen, Dauerwellen
oder Färben ausgesetzt worden ist.

/3

0005805
HENKEL KGaA
ZR-FE/Patente

Die erfindungsgemäß in den haarkosmetischen Mitteln zu verwendende Borsäure kann in Mengen von 0,5 bis 8 Gewichtsprozent, vorzugsweise 1 - 5 Gewichtsprozent, bezogen auf das gesamte Mittel, eingesetzt werden. Daneben können die Mittel grenzflächenaktive Verbindungen, Verdickungsmittel, Kunstharze, Weichmacher, Antischuppenmittel, Konservierungsstoffe, Parfüms, Lösungsmittel wie niedere Alkohole und andere übliche Rezepturbestandteile enthalten. Die haarkosmetischen Mittel können als Cremes, Gele, Lotionen und in selteneren Fällen auch als Aerosole mit einem Treibmittelanteil vorliegen.

Die nachfolgenden Beispiele sollen den Erfindungsgegenstand näher erläutern, ohne ihn jedoch hierauf zu beschränken.

/4

0005805
HENKEL KGaA
ZR-FE/Patente

B e i s p i e l e

1) Haarspülbad

Kolloiddisperses Gemisch von
Cetylstearylalkohol, Natriumcetylstearylsulfat und nichtionogenen Emulgator, Emulgade F[(R)]

| | | |
|---|---|---|
| Dehydag | 3,5 | Gew.-Teile |
| Ölsäuredecylester | 10,0 | " |
| Glycerin | 3,0 | " |
| Borsäure | 2,0 | " |
| Zitronensäure | 3,0 | " |
| Parfüm | 0,5 | " |
| Wasser | 78,0 | " |

2) Haarregenerator

| | | |
|---|---|---|
| Cetylstearylalkohol | 4,0 | Gew.-Teile |
| Borsäure | 4,0 | " |
| Zitronensäure | 1,0 | " |
| Copaiva Balsamöl | 0,1 | " |
| Parfüm | 1,0 | " |
| Wasser | 89,9 | " |

3) Haarfestiger

Vinylpyrrolidon/Vinylacetat-
Copolymeres Nasuna B[(R)],

| | | |
|---|---|---|
| Dehydag | 1,5 | Gew.-Teile |
| Borsäure | 1,5 | " |
| Isopropanol | 50,0 | " |
| Parfüm | 1,0 | " |
| Wasser | 46,0 | " |

4) Birkenhaarwasser

| | | |
|---|---|---|
| Polyol-Fettsäureester, Cetiol HE$^{(R)}$ Dehydag | 2,0 | GewTeile |
| Extrapon Birke spezial | 1,0 | " |
| Borsäure | 3,0 | " |
| Isopropanol | 30,0 | " |
| Parfüm | 1,0 | " |
| Wasser | 63,0 | " |

5) Haarwasser

| | | |
|---|---|---|
| Isopropanol | 60,0 | GewTeile |
| Glycerin | 3,0 | " |
| Borsäure | 2,0 | " |
| 2,2'-Dioxy-3,5,6,3',5',6'-hexachlordiphenylmethan | 0,2 | " |
| Parfüm | 1,0 | " |
| Wasser | 33,8 | " |

6) Frisiercreme

| | | |
|---|---|---|
| Polyolfettsäureester | 8,0 | GewTeile |
| Isopropanol | 20,0 | " |
| Verdickungsmittel Carbopol 940 | 1,0 | " |
| Triäthanolamin | 0,8 | " |
| Borsäure | 4,0 | " |
| Parfüm | 1,2 | " |
| Wasser | 65,0 | " |

0005805
HENKEL KGaA
ZR-FE/Patente

"Verwendung von Borsäure zur Verbesserung der Struktur und der Naßkämmbarkeit von Haaren"

___

Patentansprüche:

1. Verwendung von Borsäure in haarkosmetischen Mitteln zur gleichzeitigen Verbesserung der Struktur und der Naßkämmbarkeit von Haaren.

2. Verwendung von Borsäure nach Anspruch 1 in einer Menge von 0,5 bis 8 Gewichtsprozent, vorzugsweise 1 bis 5 Gewichtsprozent, bezogen auf das gesamte haarkosmetische Mittel.

0005805

Europäisches Patentamt

**EUROPÄISCHER RECHERCHENBERICHT**

Nummer der Anmeldung

EP 79 10 1606

| | EINSCHLÄGIGE DOKUMENTE | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| X | <u>BE - A - 514 899</u> (SCHWARZKOPF)<br><br>* Seite 2, Zeilen 29-32; Beispiele 2,3; Patentansprüche 1,2 *<br><br>-- | 1,2 | A 61 K 7/06<br>7/08<br>7/11 |
| X | CHEMICAL ABSTRACTS, Band 74, Nr. 26, veröff. am 28-06-1971 Columbus, Ohio, USA Seite 302, Spalte 2, Zusammenfassung 146261k<br><br>& CS - A - 135 889 (O. FERTET et al. ) veröff. am 15-03-1970.<br><br>* Zusammenfassung *<br><br>-- | 1,2 | |
| X | <u>GB - A - 993 967</u> (SUPERMA LTD.)<br><br>* Beispiel 1 *<br><br>-- | 1,2 | **RECHERCHIERTE SACHGEBIETE (Int.Cl.²)**<br><br>A 61 K 7/06<br>7/08<br>7/11<br>7/09 |
| X | <u>CH - A - 153 325</u> (CORIOLAN GmbH.)<br><br>* Beispiel B; Seite 2, rechte Spalte, Zeilen 1-5 *<br><br>-- | 1 | |
| | <u>US - A - 2 290 908</u> (H. GUNNING)<br><br>* Patentanspruch *<br><br>-- | 1 | |
| | CHEMICAL ABSTRACTS, Band 88, Nr. 6, veröff. am 06-02-1978, Columbus, Ohio, USA Seite 241, Spalte 2, Zusammenfassung 41544r<br><br>& JP - A - 77 58709 (M. OKUDA) Veröff. am 14-05-1979.<br><br>* Zusammenfassung *<br>-- | 1 | |

./.

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 04-09-1979 | WILLEKENS |

EPA form 1503.1 06.78

## EUROPÄISCHER RECHERCHENBERICHT

Europäisches
Patentamt

| | EINSCHLÄGIGE DOKUMENTE | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | FR - A - 698 074 (M. VISTARINI)<br>* Zusammenfassung 1 und 2 *<br><br>-- | 1,2 | |
| | FR - A - 865 747 (I.G. FARBEN)<br>* Beispiel 1; Zusammenfassung *<br><br>-- | 1 | |
| | FR - A - 2 357 569 (CIBA GEIGY)<br>* Seite 7, Zeilen 8-12; Patent-<br>ansprüche 1,16 *<br><br>---- | 1 | RECHERCHIERTE SACHGEBIETE (Int. Cl.²) |